# EUROPEAN PATENT SPECIFICATION

(11) **EP 4 081 109 B1**
(45) Date of publication and mention of the grant of the patent: **09.09.2026**
(21) Application number: 21763554.9
(22) Date of filing: 03.03.2021
(51) Int. Cl.: A61B 5/00, A61B 5/276, A61B 5/332, A61B 5/349, A61B 5/053

(54) **WEARABLE ELECTRONIC DEVICE**
ELEKTRONISCHE WEARABLE-VORRICHTUNG
DISPOSITIF ÉLECTRONIQUE PORTABLE

(30) Priority: 05.03.2020 KR 20200027878
(43) Date of publication of application: 02.11.2022
(73) Proprietor: Samsung Electronics Co., Ltd., Suwon-si, Gyeonggi-do 16677 (KR)
(72) Inventor: CHO, Minhyun, Suwon-si Gyeonggi-do 16677 (KR); JUNG, Hyunjun, Suwon-si Gyeonggi-do 16677 (KR); KIM, Younghyun, Suwon-si Gyeonggi-do 16677 (KR); KIM, Taehyeon, Suwon-si Gyeonggi-do 16677 (KR); LIM, Daehyeong, Suwon-si Gyeonggi-do 16677 (KR); PARK, Jeongmin, Suwon-si Gyeonggi-do 16677 (KR)
(74) Representative: Gulde & Partner
(86) International application number: PCT/KR2021/002632
(87) International publication number: WO 2021/177725

(56) References cited:
- EP-A1- 3 153 099
- WO-A2-2017/182677
- JP-A- 2012 191 995
- JP-A- 2017 121 442
- KR-A- 20190 097 474
- US-A1- 2014 243 643
- US-A1- 2016 074 674
- US-A1- 2017 181 644

## Description

### [Technical Field]

Embodiments of the present disclosure relate to a wearable electronic device with biometric detection capability, and more particularly, to improving the accuracy of biometric detection in wearable devices.

### [Background Art]

Wearable electronic devices have become increasingly widespread, and demand has grown for wearable electronic devices that incorporate increasingly diverse functionality.

In particular, because wearable electronic devices have high usability and portability, biometric detection functions have been added, and are growing in use and sophistication. One function may include health care services integrating with detection of biometric information using the wearable electronic device. The development of increasingly accurate biometric information sensing methods will serve to increase the utility and benefit of these health care services.

US 2017/181644 A1 describes a wearable device which has electrical contacts integrated in a housing base of the wearable device as electrodes designed to contact skin of a user while wearing the wearable device. A housing bezel of the wearable device is designed as an additional point of contact on the wearable device. The wearable device includes an electromyography (EMG) system to receive electrical signals from at least two of the electrodes and detect muscular movement of the user. Further, the wearable device includes an electrocardiogram (ECG) system to receive and combine the electrical signals from the electrodes when the user touch contacts the housing bezel while wearing the wearable device to complete an ECG loop between the electrodes and the housing bezel for a heart rate reading of the user.

WO 2017/182677 A2 describes a wearable device for measuring cardiac activity of a user, the wearable device comprising: an optical cardiac activity sensor unit configured to be placed in contact with a measurement area and to enable cardiac activity measurement of the user to obtain a cardiac activity signal; a plurality of electrodes configured to enable bioimpedance measurement on the measurement area to obtain a bioimpedance signal; means for detecting changes in the bioimpedance signal; and means for reducing a motion artefact effect on the cardiac activity signal based on the detected changes in the bioimpedance signal.

### [Disclosure]

### [Technical Problem]

When detecting biometric information using a wearable electronic device, the accuracy of the detected biometric information may be reduced based on contours, structures and other features of a body part on which the wearable electronic device is worn. Other factors which affect accuracy may include the state of skin contacting the wearable electronic device, and an external environment at present location of the device and the user.

Accordingly, an aspect of the present disclosure is to provide a wearable electronic device with increased biometric detection accuracy, by accounting for the influence of external factors on detection, such as the user's body, the skin condition and the external environment, and thus provide heightened accuracy in biometric information.

### [Technical Solution]

In accordance with an aspect of the present disclosure, a wearable electronic device is provided. The wearable electronic device includes a housing including a front plate and a rear plate, three or more rear-side electrodes disposed on the rear plate, a biometric signal processing circuit disposed within the housing, and a processor disposed within the housing and operatively connected to the biometric signal processing circuit, wherein the processor is configured to: based on first information, execute an electrode combining operation by setting at least one rear-side electrode as a first electrode set, and setting at least one other rear-side electrode as a second electrode set, determine whether to execute a recombination operation of the three or more rear-side electrodes based on second information, and detect biometric information using the first electrode set and the second electrode set.

In accordance with another aspect of the present disclosure, a wearable electronic device is provided. The wearable electronic device includes a housing including a front plate and a rear plate, three or more rear-side electrodes disposed on the rear plate, a biometric signal processing circuit disposed within the housing, an other-side electrode located on one side of the housing and electrically connected to the biometric signal processing circuit, and a processor disposed within the housing, wherein the processor is configured to detect a DC offset using at least one of the three or more rear-side electrodes and the other-side electrode, select at least two rear-side electrodes from among the three or more rear-side electrodes based on the DC offset, operably connect the at least two selected rear-side electrodes to the biometric signal processing circuit, and obtain biometric information using the at least two rear-side electrodes connected to the biometric signal processing circuit and the other-side electrode.

### [Advantageous Effects]

According to embodiments, a wearable electrode device may measure accurate biometric information by reducing an influence of a user's wearing state on a biometric information measurement result.

### [Description of Drawings]

FIG. 1 is a block diagram illustrating an electronic device in a network according to certain embodiments.
FIG. 2 is a block diagram illustrating a wearable electronic device according to an embodiment.
FIG. 3 is a configuration diagram illustrating a wearable electronic device according to an embodiment.
FIG. 4 is a planar view illustrating a wearable electronic device according to an embodiment.
FIG. 5 is a flowchart illustrating operation of a wearable electronic device according to an embodiment.
FIG. 6 is a graph illustrating a variation in light quantity over time, detected by an optical sensor of a wearable electronic device according to an embodiment.
FIG. 7 is a graph illustrating a DC offset over time, detected by a wearable electronic device according to an embodiment.
FIG. 8 is a planar view illustrating a wearable electronic device according to an embodiment.
FIG. 9 is a planar view illustrating a wearable electronic device according to an embodiment.

With respect to the description of the drawings, the same or similar reference signs may be used for the same or similar elements.

### [Mode for Invention]

Hereinafter, certain embodiments of the present disclosure will be described in detail with reference to the accompanying drawings. However, it should be understood that the present disclosure is not limited to specific embodiments, but rather includes various modifications, equivalents and/or alternatives of certain embodiments of the present disclosure.

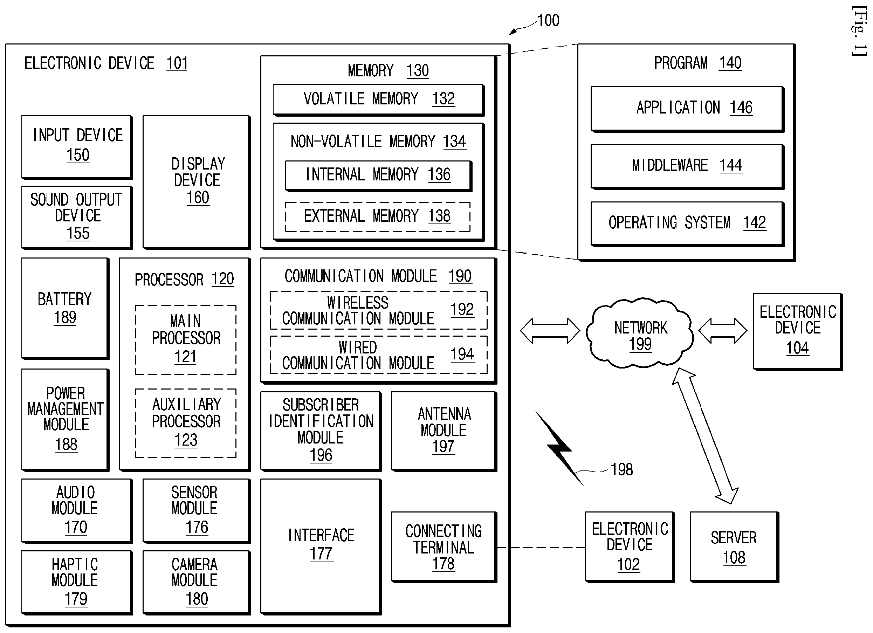

Fig. 1 is a block diagram illustrating an electronic device 101 in a network environment 100 according to certain embodiments. Referring to Fig. 1, the electronic device 101 in the network environment 100 may communicate with an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input device 150, a sound output device 155, a display device 160, an audio module 170, a sensor module 176, an interface 177, a haptic module 179, a camera module 180, a power management module 188, a battery 189, a communication module 190, a subscriber identification module (SIM) 196, or an antenna module 197. In some embodiments, at least one (e.g., the display device 160 or the camera module 180) of the components may be omitted from the electronic device 101, or one or more other components may be added in the electronic device 101. In some embodiments, some of the components may be implemented as single integrated circuitry. For example, the sensor module 176 (e.g., a fingerprint sensor, an iris sensor, or an illuminance sensor) may be implemented as embedded in the display device 160 (e.g., a display).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may load a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), and an auxiliary processor 123 (e.g., a graphics processing unit (GPU), an image signal processor (ISP), a sensor hub processor, or a communication processor (CP)) that is operable independently from, or in conjunction with, the main processor 121. Additionally or alternatively, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display device 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input device 150 may receive a command or data to be used by other component (e.g., the processor 120) of the electronic device 101, from the outside (e.g., a user) of the electronic device 101. The input device 150 may include, for example, a microphone, a mouse, a keyboard, or a digital pen (e.g., a stylus pen).

The sound output device 155 may output sound signals to the outside of the electronic device 101. The sound output device 155 may include, for example, a speaker or a receiver. The speaker may be used for general purposes, such as playing multimedia or playing record, and the receiver may be used for an incoming calls. According to an embodiment, the receiver may be implemented as separate from, or as part of the speaker.

The display device 160 may visually provide information to the outside (e.g., a user) of the electronic device 101. The display device 160 may include, for example, a display, a hologram device, or a projector and control circuitry to control a corresponding one of the display, hologram device, and projector. According to an embodiment, the display device 160 may include touch circuitry adapted to detect a touch, or sensor circuitry (e.g., a pressure sensor) adapted to measure the intensity of force incurred by the touch.

The audio module 170 may convert a sound into an electrical signal and vice versa. According to an embodiment, the audio module 170 may obtain the sound via the input device 150, or output the sound via the sound output device 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wirelessfidelity (Wi-Fi) direct, or infrared data association (IrDA)) or the second network 199 (e.g., a long-range communication network, such as a cellular network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The antenna module 197 may transmit or receive a signal or power to or from the outside (e.g., the external electronic device) of the electronic device 101. According to an embodiment, the antenna module 197 may include an antenna including a radiating element implemented using a conductive material or a conductive pattern formed in or on a substrate (e.g., PCB). According to an embodiment, the antenna module 197 may include a plurality of antennas. In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 and 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, or client-server computing technology may be used, for example.

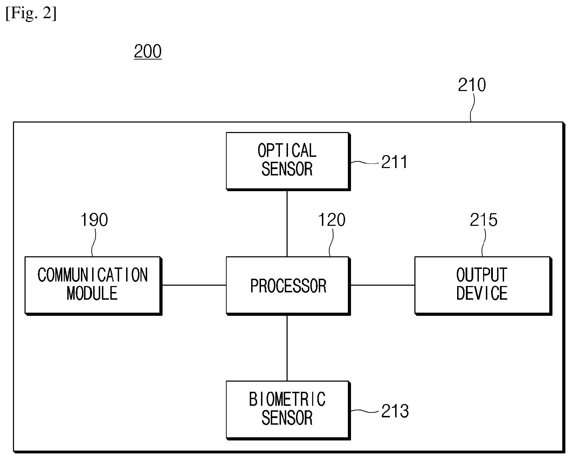

Hereinafter, a wearable electronic device according to an embodiment will be described with reference to FIG. 2. FIG. 2 is a block diagram 200 illustrating a wearable electronic device according to an embodiment.

Referring to FIG. 2, a wearable electronic device 210 may include a processor 120, an optical sensor 211, a biometric sensor 213, a communication module 190, and an output device 215.

The processor 120 may be electrically or operatively coupled to the other elements (e.g., at least one of the optical sensor 211, the biometric sensor 213, the communication module 190, or the output device 215) of the wearable electronic device 210, and may be configured to control the other elements of the wearable electronic device 210. In the embodiments described below, operation of the wearable electronic device 210 may be referred to as operation of the processor 120.

The output device 215 may include a display. According to an embodiment, the display may be configured to provide visual information to a user and receive a user input (e.g., a touch input). According to an embodiment, the output device 215 may include an indicator (e.g., at least one light-emitting diode (LED)). The indicator may be configured to emit light having at least one wavelength through a front side (e.g., the surface in which the display of the wearable electronic device 210 is positioned) of a housing of the wearable electronic device 210 and/or a side surface thereof. For example, the wearable electronic device 210 may provide a notification to the user using the output device (e.g., a display and/or indicator). For another example, the wearable electronic device 210 may provide a sound notification to the user using a speaker.

The communication module 190 may be configured to communicate with an external electronic device (e.g., the electronic device 102, the electronic device 104, or the server 108 of FIG. 1) via various networks (e.g., the second network 199 and/or the first network 198 of FIG. 1). According to an embodiment, the processor 120 may transmit information associated with the wearable electronic device 210 to the external electronic device or receive information from the external electronic device using the communication module 190. For example, the wearable electronic device 210 may use the communication module 190 to transmit, to the external electronic device, data sensed by the biometric sensor 213 or associated data obtained based on the sensed data.

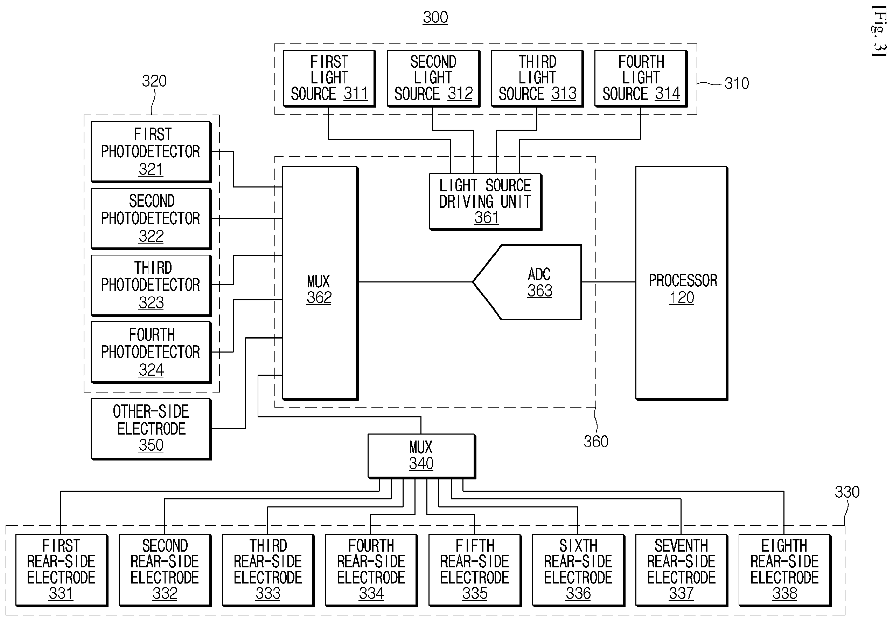

The optical sensor 211 may include a plurality of photodetectors 320 (see FIG. 3), a light source 310 (see FIG. 3), and an optical signal processing circuit (not shown). The processor 120 may obtain light quantity information based on light quantities detected by each of the plurality of photodetectors 320 using the optical sensor 211.

The biometric sensor 213 may include a plurality of rear-side electrodes 330 (see FIG. 3), an "other-side" electrode 350 (see FIG. 3), and a biometric signal processing circuit (not shown). The processor 120 may obtain biometric information using the biometric sensor 213. For example, the biometric information may include heart rate information or atrial fibrillation information based on electrocardiogram (ECG). For another example, the biometric information may include body composition information, body fat information, or body moisture information based on bioelectrical impedance analysis (BIA). For another example, the biometric information may include skin moisture level information based on galvanic skin response (GSR).

Hereinafter, a wearable electronic device (e.g., the wearable electronic device 210 of FIG. 2) according to an embodiment will be described with reference to FIG. 3. FIG. 3 is a configuration diagram 300 illustrating a wearable electronic device according to an embodiment.

The wearable electronic device according to an embodiment may include the light source 310, the plurality of photodetectors 320, the plurality of rear-side electrodes 330, the other-side electrode 350, a sensor driving circuit 360, and the processor 120.

The light source 310 may include a first light source 311, a second light source 312, a third light source 313, and a fourth light source 314. Although FIG. 3 illustrates four light sources, the number of light sources is not limited thereto. The light source 310 may include at least one light-emitting element (e.g., a light-emitting diode (LED)) for radiating light having a wavelength within a specified range. For example, each light source may be configured to emit light of different wavelengths. For another example, at least a portion of the light source 310 may be configured to emit light of the same wavelength.

Each of the plurality of photodetectors 320 may detect light and determine an intensity of the detected light. For example, each of the plurality of photodetectors 320 may output a current signal having a magnitude corresponding to a detected light quantity. The plurality of photodetectors 320 may include a first photodetector 321, a second photodetector 322, a third photodetector 323, and a fourth photodetector 324. The first photodetector 321, the second photodetector 322, the third photodetector 323, and the fourth photodetector 324 may be connected to the processor 120 via a multiplexer (MUX) 362. Although FIG. 3 illustrates four photodetectors, the number of photodetectors is not limited thereto.

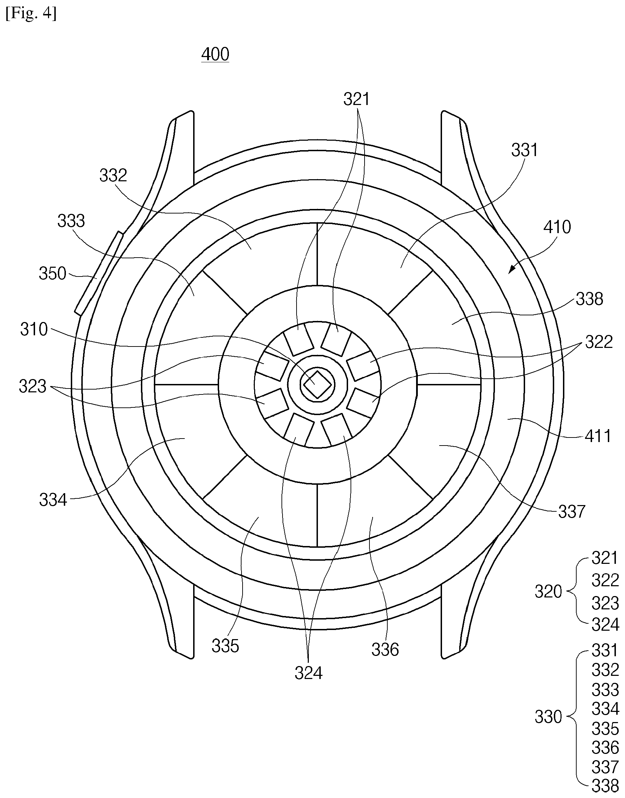

The plurality of rear-side electrodes 330 and the other-side electrode 350 may contact a portion of a user's body so as to be used to detect biometric information. The plurality of rear-side electrodes 330 may be connected to the sensor driving circuit 360 via a MUX 340. The plurality of rear-side electrodes 330 may include a first rear-side electrode 331, a second rear-side electrode 332, a third rear-side electrode 333, a fourth rear-side electrode 334, a fifth rear-side electrode 335, a sixth rear-side electrode 336, a seventh rear-side electrode 337, and an eighth rear-side electrode 338. Although FIG. 3 illustrates eight rear-side electrodes, the number of rear-side electrodes is not limited thereto. The plurality of rear-side electrodes 330 may include three or more rear-side electrodes. The plurality of rear-side electrodes 330 may be arranged on one surface of a housing 410 (see FIG. 4), and the other-side electrode 350 may be arranged on another surface of the housing 410. For example, the plurality of rear-side electrodes 330 may be arranged on a rear surface of the housing 410, and the other-side electrode 350 may be arranged on a side surface or front surface of the housing 410.

At least one of the plurality of rear-side electrodes 330 may be included in a first electrode set, and at least one of the plurality of rear-side electrodes 330 except for the first electrode set may be included in a second electrode set. The first electrode set 330 and the other-side electrode 350 may collect biometric information at different positions, and the second electrode set 330 may be used for biological bias and in-phase component noise reduction. For example, when worn by the user, the first electrode set 330 and the second electrode set 330 may contact skin of a wrist, and the other-side electrode 350 may contact a finger of the other hand.

The sensor driving circuit 360 may include a light source driving unit 361, the MUX 362, and an analog-to-digital converter (ADC) 363. The sensor driving circuit 360 may further include other elements (e.g., an amplifier, a filter, and/or a memory) not illustrated in FIG. 3. The sensor driving circuit 360 may be electrically connected to the processor 120, and may operate as an interface and/or hub between various sensors and the processor 120.

The light source driving unit 361 may control the light source 310 in a specified state. The processor 120 may control the light source 310 using the light source driving unit 361. For example, the light source driving unit 361 may control the light source 310 so that the light source 310 may emit light having a specified wavelength. The light source driving unit 361 may control the light source 310 so that the light source 310 may emit light for a specified time. The light source driving unit 361 may control the light source 310 so that the light source 310 may emit light a specified number of times.

The ADC 363 may convert analog signals sensed by the plurality of photodetectors 320, the plurality of rear-side electrodes 330, and the other-side electrode 350 into digital signals. The signals converted by the ADC 363 may be transferred to the processor 120.

The processor 120 may be configured to detect whether the first electrode set 330, the second electrode set 330, and the other-side electrode 350 are in contact with a body via the first electrode set 330, the second electrode set 330, and the other-side electrode 350. For example, the processor 120 may determine whether the first electrode set 330, the second electrode set 330 and the other-side electrode 350 are in contact with a body based on an output value from a contact detection module (e.g., a comparator) according to an input of a voltage applied through a living body or a voltage applied from a voltage source.

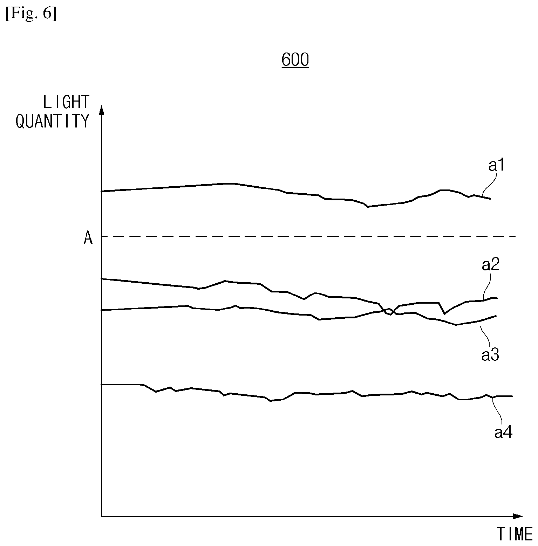

The processor 120 may be configured to determine whether the plurality of rear-side electrodes 330 are not in full contact with a user's body and thus in a "floating" state relative to the same (i.e., "floated), by obtaining light quantity information based on light quantities detected by each of the plurality of photodetectors 320 using an optical sensor. For example, when a rear-side electrode of the rear-side electrodes 330 is floated, additional light may be detected by at least one of the plurality of photodetectors 320, which is adjacent to the rear-side electrode in the floated state. For example, light emitted from the light source 310 and/or external light may be detected through the at least one of photodetectors 320, which is adjacent to the rear-side electrode in the floated state. Therefore, a light quantity which exceeds a maximum threshold light quantity "A" (e.g., see FIG. 6) is detected, the processor 120 may determine that one or more of the rear-side electrodes 330 are in the floated state.

The processor 120 may be configured to select some electrodes for obtaining biometric information from among the plurality of rear-side electrodes connected to the single MUX 340. The processor 120 may perform rear-side electrodes combining (e.g., combining subsets of the rear-side electrodes into a first and second electrode set) based on first information. Here, the first information may include the light quantity information. That is, the processor 120 may select, as the first electrode set 330, at least one rear-side electrode from among the plurality of rear-side electrodes 330 based on the light quantity information, and may select, as the second electrode set 330, at least one rear-side electrode from among the plurality of rear-side electrodes 330 except for the first electrode set 330. The first electrode set 330 and the second electrode set 330 may be electrically connected to the biometric signal processing circuit. For example, the processor 120 may generate an instruction for electrically connecting, to the biometric signal processing circuit, the rear-side electrodes 330 except for a rear-side electrode located in a region adjacent to the at least one of the plurality of photodetectors 320 in which a light quantity exceeding the maximum light quantity A (see FIG. 6) has been detected. The processor 120 may select the rear-side electrodes 330 except for the rear-side electrode 330 located within a first distance from the at least one of the plurality of photodetectors 320 in which a light quantity exceeding the maximum light quantity A (see FIG. 6) has been detected, so as to include the selected rear-side electrodes 330 in the first electrode set and the second electrode set.

The processor 120 may be configured to detect whether biometric information detected by the first electrode set 330, the second electrode set 330, and the other-side electrode 350 is valid. Whether or not the biometric information is valid may be determined by measuring a DC offset value that is a difference between DC voltages on the first electrode set 330 and the other-side electrode 350.

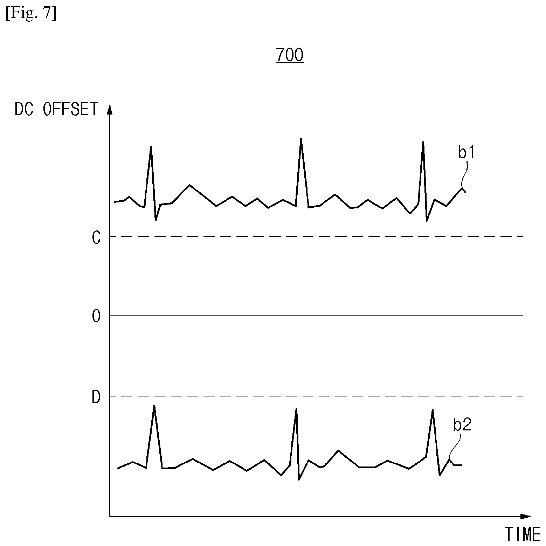

If the DC offset does not have a value that is sufficiently close to 0, an amplified biometric signal may deviate from an operation range (e.g., for operating a biometric sensor). Therefore, the DC offset may be implemented using a value that is sufficiently close to 0 in order to detect accurate biometric information. For example, the DC offset may have a value within a first range between a first voltage value C (see FIG. 7) and a second voltage value D (see FIG. 7).

The processor 120 may determine whether to perform rear-side electrodes recombining based on second information. The second information may include DC offset information. When the value of the DC offset does not fall within the first range between the first voltage value C (see FIG. 7) and the second voltage value D (see FIG. 7), the processor 120 may perform a rear-side electrode recombining operation, to reselect the first electrode set and the second electrode set. The processor 120 may be configured to determine, according to the DC offset, the number of rear-side electrodes to be included in the first electrode set 330, the number of rear-side electrodes to be included in the second electrode set 330, or arrangement of the first electrode set 330 and the second electrode set 330. For example, if contact impedance on a side of the first electrode set 330 is relatively low, a DC voltage on the first electrode set 330 increases, and thus the DC offset that is a DC voltage difference between the first electrode set 330 and the other-side electrode 350 may be high. Furthermore, if the contact impedance on the side of the first electrode set 330 is relatively high, the DC voltage on the first electrode set 330 decreases, and thus the DC offset that is a DC voltage difference between the first electrode set 330 and the other-side electrode 350 may be low. Therefore, if the DC offset is higher than the first voltage value C (see FIG. 7), the wearable electronic device according to an embodiment may reduce the number of rear-side electrodes included in the first electrode set 330 connected to the biometric signal processing circuit. In this case, the contact impedance of the first electrode set 330 may be increased so that the DC offset may be decreased to a value that is close to 0. On the contrary, if the DC offset is lower than the second voltage value D (see FIG. 7), the wearable electronic device according to an embodiment may increase the number of rear-side electrodes included in the first electrode set 330 connected to the biometric signal processing circuit. In this case, the contact impedance of the first electrode set 330 may be decreased so that the DC offset may be increased to a value that is close to 0.

The processor 120 may be configured to obtain biometric information through the first electrode set 330, the second electrode set 330, and the other-side electrode 350 when the value of the DC offset falls within the first range between the first voltage value C (see FIG. 7) and the second voltage value D (see FIG. 7).

Since the wearable electronic device according to an embodiment may detect biometric information by selectively using the rear-side electrode 330 according to measured light quantity information and DC offset, an influence of a user's wearing state on biometric information may be reduced, and accurate biometric information may be obtained.

Hereinafter, a planar structure of a wearable electronic device according to an embodiment will be described with reference to FIG. 4. FIG. 4 is a planar view 400 illustrating a wearable electronic device according to an embodiment.

A wearable electronic device (e.g., the wearable electronic device of FIG. 2) according to an embodiment may include the housing 410, the light source 310, the plurality of photodetectors 320, the plurality of rear-side electrodes 330, and the other-side electrode 350.

The housing 410 may include a front plate (not shown) and a rear plate 411. The housing 410 may surround and protect elements included in the wearable electronic device or may fixe some elements.

The light source 310 may be located on the rear plate 411 of the housing 410 to emit light in a direction to a rear side of the housing 410. Although FIG. 4 illustrates the light source 310 as being located at a center of the rear plate 411 of the housing 410, a location of the light source 310 is not limited thereto and may be located on any portion of the rear plate 411.

The plurality of photodetectors 320 may be located on the rear plate 411 of the housing 410. The plurality of photodetectors 320 may include the first photodetector 321, the second photodetector 322, the third photodetector 323, and the fourth photodetector 324. The first photodetector 321, the second photodetector 322, the third photodetector 323, and the fourth photodetector 324 may surround the light source 310. The plurality of photodetectors 320 may be disposed adjacent to the plurality of rear-side electrodes 330. In other words, each of the plurality of photodetectors 320 may be located at a distance, shorter than a first threshold distance, from at least one of the plurality of rear-side electrodes 330.

The plurality of rear-side electrodes 330 may be located on the rear plate 411 of the housing 410. The plurality of rear-side electrodes 330 may include the first rear-side electrode 331, the second rear-side electrode 332, the third rear-side electrode 333, the fourth rear-side electrode 334, the fifth rear-side electrode 335, the sixth rear-side electrode 336, the seventh rear-side electrode 337, and the eighth rear-side electrode 338. The first rear-side electrode 331, the second rear-side electrode 332, the third rear-side electrode 333, the fourth rear-side electrode 334, the fifth rear-side electrode 335, the sixth rear-side electrode 336, the seventh rear-side electrode 337, and the eighth rear-side electrode 338 may surround the plurality of photodetectors 320. However, a location of the plurality of rear-side electrodes 330 is not limited thereto, and the plurality of rear-side electrodes 330 may be located in other regions on the rear plate 411. The first rear-side electrode 331, the second rear-side electrode 332, the third rear-side electrode 333, the fourth rear-side electrode 334, the fifth rear-side electrode 335, the sixth rear-side electrode 336, the seventh rear-side electrode 337, and the eighth rear-side electrode 338 may be spaced apart from each other. At least one of the first rear-side electrode 331, the second rear-side electrode 332, the third rear-side electrode 333, the fourth rear-side electrode 334, the fifth rear-side electrode 335, the sixth rear-side electrode 336, the seventh rear-side electrode 337, and the eighth rear-side electrode 338 may be selected by the processor 120 (see FIG. 3) and may be included in the first electrode set connected to the biometric signal processing circuit. At least one of the first rear-side electrode 331, the second rear-side electrode 332, the third rear-side electrode 333, the fourth rear-side electrode 334, the fifth rear-side electrode 335, the sixth rear-side electrode 336, the seventh rear-side electrode 337, and the eighth rear-side electrode 338 except for the first electrode set may be selected by the processor 120 (see FIG. 3) and may be included in the second electrode set connected to the biometric signal processing circuit.

The other-side electrode 350 may be located on one side of the housing 410. For example, the other-side electrode 350 may be located on a side surface of the housing 410. For another example, the other-side electrode 350 may be located on a front side of the housing 410. The other-side electrode 350 may be connected to the biometric signal processing circuit to serve to detect biometric information together with the first electrode set and the second electrode set.

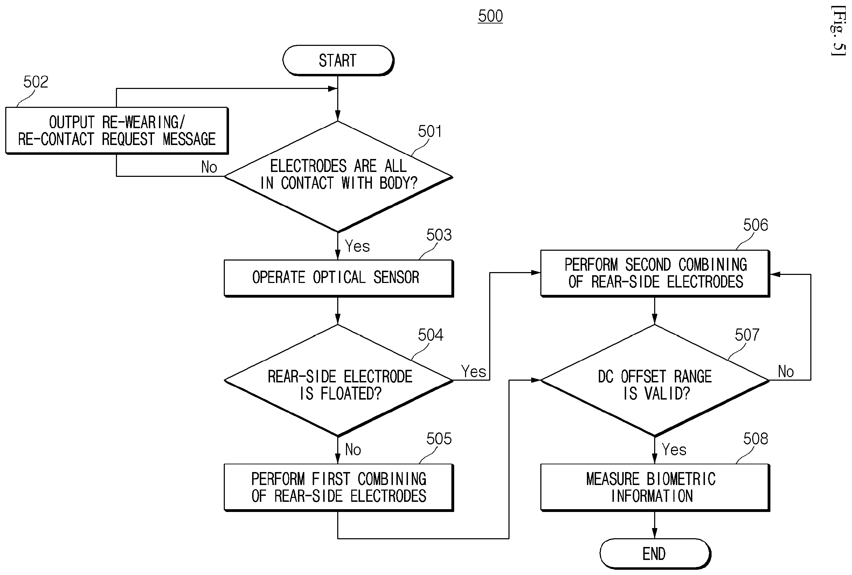

Hereinafter, operation of a wearable electronic device according to an embodiment will be described with reference to FIG. 5. FIG. 5 is a flowchart 500 illustrating operation of a wearable electronic device according to an embodiment.

Referring to FIG. 5, in operation 501, a wearable electronic device according to an embodiment may determine whether all of the first electrode set, the second electrode set, and the other-side electrode are in contact with a body of a user. Specifically, the wearable electronic device according to an embodiment may determine whether at least a first number of rear-side electrodes of the first electrode set, at least a second number of rear-side electrodes of the second electrode set, and the other-side electrode are all in contact with the body. For example, contact of a body with the first electrode set 330 (see FIG. 3), the second electrode set 330 (see FIG. 3), and/or the other-side electrode 350 may be detected by applying a DC voltage on an electrode to a comparator circuit embedded in an analog front end (AFE) and obtaining a value thereof.

In operation 502, if it is determined that at least one of the first electrode set (e.g., or at least the first number of rear-side electrodes of the first electrode set), the second electrode set (e.g., or at least the second number of rear-side electrodes of the second electrode set), or the other-side electrode is not in contact with a body, the wearable electronic device according to an embodiment may output a prompt (e.g., a "re-wearing request message") requesting the user to reposition or attempt remounting of the wearable electronic device on their body. Furthermore, the wearable electronic device according to an embodiment may output a prompt (e.g., a "re-contact request message") requesting repositioning of the device to initiate contact with the body, if it is determined that the other-side electrode is not in contact with a body.

In operation 503, an optical sensor may be activated if it is determined that all of the first electrode set (i.e., or at least the first number of rear-side electrodes of the first electrode set), the second electrode set (i.e., or at least the second number of rear-side electrodes of the second electrode set), and the other-side electrode are in contact with a body. In detail, the light source 310 (see FIG. 3) may emit light, and light quantity information as reflected from the user's body may be obtained through the plurality of photodetectors 320 (see FIG. 3). According to an embodiment, operation 503 may be skipped.

In operation 504, the wearable electronic device according to an embodiment may determine whether at least one of rear-side electrodes is "floating" (e.g., not contacting or insufficiently contacting the user's body) based on the light quantity information. In detail, the wearable electronic device according to an embodiment may determine whether the light quantities detected by each of the plurality of photodetectors 320 (see FIG. 3) exceeds a maximum light quantity A (see FIG. 6). When the light quantity detected by a photodetector exceeds maximum light quantity A, the wearable electronic device according to an embodiment may determine that at least one rear-side electrode located within a first distance from the photodetector is in the floated-state (e.g., or "floated" for short).

In operation 505, the wearable electronic device according to an embodiment may perform a first combination of rear-side electrodes if it is determined that none of the rear-side electrodes are floated. That is, at least one of the plurality of rear-side electrodes may be selected as the first electrode set, and one or more other rear-side electrodes may be selected as the second electrode set. The selected first electrode set and second electrode set may be connected to the biometric signal processing circuit. Here, a first count of rear-side electrodes included in the first electrode set and a second count of rear-side electrodes included in the second electrode set may be equal to the total number of the plurality of rear-side electrodes. In other words, as a result of the first combining of rear-side electrodes, all of the plurality of rear-side electrodes may be connected to the biometric signal processing circuit.

In operation 506, the wearable electronic device according to an embodiment may perform second combining of rear-side electrodes if it is determined that there is a floated rear-side electrode. For example, the wearable electronic device according to an embodiment may operably connect the plurality of rear-side electrodes to the biometric signal processing circuit, except for the rear-side electrode that is adjacent to a photodetector, for which the light quantity exceeds the maximum light quantity A (see FIG. 6) (e.g., detected as floated). The operably connected subset of rear-side electrodes may be partitioned into the first electrode set and the second electrode set. Here, the sum of the number of rear-side electrodes included in the first electrode set and the number of rear-side electrodes included in the second electrode set may be less than the total number of the plurality of rear-side electrodes. In other words, as a result of the second combining of rear-side electrodes, at least one of the plurality of rear-side electrodes is excluded from being connected to the biometric signal processing circuit, due to being floated. In some embodiments additional rear-side electrodes may be excluded from the second combination operation as well.

In operation 507, the wearable electronic device according to an embodiment may determine whether a DC offset range is valid. For example, the wearable electronic device according to an embodiment may determine whether the DC offset has a value within a first predefined range (e.g., between the first voltage value C (see FIG. 7) and the second voltage value D (see FIG. 7)).

If it is determined that the DC offset range is not valid, the wearable electronic device according to an embodiment may re-perform the second combining operation of rear-side electrodes of operation 506. If the DC offset is higher than the first voltage value C (see FIG. 7), the number of rear-side electrodes included in the first electrode set connected to the biometric signal processing circuit may be reduced. On the contrary, if the DC offset is lower than the second voltage value D (see FIG. 7), the number of rear-side electrodes included in the first electrode set connected to the biometric signal processing circuit may be increased. That is, in embodiments where some non-floated rear-side electrodes were not included in the combining operations, the wearable electronic device according to an embodiment may select additional non-combined rear-side electrodes from among the plurality of rear-side electrodes based on the DC offset, and may add them to the combination of rear-side electrodes operably connected to the biometric signal processing circuit.

According to an embodiment, the wearable electronic device may reduce or increase the number of rear-side electrodes included in the second electrode set according to a change in the number of rear-side electrodes included in the first electrode set. It has been described that the number of rear-side electrodes included in the first electrode set or the second electrode set is reduced or increased if it is determined that the DC offset range is not valid, but it is also possible to change arrangement of the first electrode set and the second electrode set. Here, combining may be performed using rear-side electrodes except for a rear-side electrode (or rear-side electrodes) determined to be floated. For example, if it is determined that two rear-side electrodes are floated among eight rear-side electrodes, the second combining may be performed using the six rear-side electrodes other than the two rear-side electrodes determined to be floated.

In operation 508, if it is determined that the DC offset range is valid, the wearable electronic device according to an embodiment may measure biometric information using the first electrode set, the second electrode set, and the other-side electrode.

Hereinafter, a method for determining whether a rear-side electrode is floated (i.e., in the floating state) using light quantity information will be described with reference to FIGS. 4 and 6. FIG. 6 is a graph 600 illustrating a variation in light quantity over time, detected by an optical sensor of a wearable electronic device according to an embodiment.

Referring to FIGS. 4 and 6, a1 indicates a light quantity detected by the first photodetector 321, a2 indicates a light quantity detected by the second photodetector 322, a3 indicates a light quantity detected by the third photodetector 323, and a4 indicates a light quantity detected by the fourth photodetector 324.

The light quantity a1 detected by the first photodetector 321 may exceed the maximum light quantity A, and the light quantity a2 detected by the second photodetector 322, the light quantity a3 detected by the third photodetector 323, and the light quantity a4 detected by the fourth photodetector 324 may be equal to or less than the maximum light quantity A. In this case, the wearable electronic device according to an embodiment may determine that floating is detected a region adjacent to the first photodetector 321 corresponding to the light quantity a1. In other words, the wearable electronic device according to an embodiment may determine that the first rear-side electrode 331 and the second rear-side electrode 332 located within the first distance from the first photodetector 321 are floated. If it is determined that floating occurs in a region adjacent to the first photodetector 321, the wearable electronic device may exclude the first rear-side electrode 331 and the second rear-side electrode 332 from connection/combination, which are adjacent to the first photodetector 321, to the biometric signal processing circuit. That is, the wearable electronic device may select the first electrode set and the second electrode set from among the third rear-side electrode 333, the fourth rear-side electrode 334, the fifth rear-side electrode 335, the sixth rear-side electrode 336, the seventh rear-side electrode 337, and the eighth rear-side electrode 338 other than the first rear-side electrode 331 and the second rear-side electrode 332.

A method for determining whether a DC offset range is valid will be described with reference to FIG. 7. FIG. 7 is a graph 700 illustrating a DC offset over time, detected by a wearable electronic device according to an embodiment. In the graph, b1 indicates that the DC offset is higher than the first voltage value C, and b2 indicates that the DC offset is lower than the second voltage value D.

If the DC offset value of b1 is detected, the wearable electronic device according to an embodiment may reduce the number of rear-side electrodes included in the first electrode set connected to the biometric signal processing circuit. That is, at least one of the rear-side electrodes included in the first electrode set may be disconnected from the biometric signal processing circuit. If the number of rear-side electrodes of the first electrode set connected to the biometric signal processing circuit is reduced, a contact area between a body and the first electrode set may be reduced, thus increasing the contact impedance and decreasing the DC offset.

If the DC offset value of b2 is detected, the wearable electronic device according to an embodiment may increase the number of rear-side electrodes included in the first electrode set connected to the biometric signal processing circuit. That is, at least one of the rear-side electrodes not presently connected to the biometric signal processing circuit as a member of the set may be added and connected to the biometric signal processing circuit. If the number of rear-side electrodes of the first electrode set connected to the biometric signal processing circuit is increased, the contact area between a body and the first electrode set may increase, thus decreasing the contact impedance and increasing the DC offset.

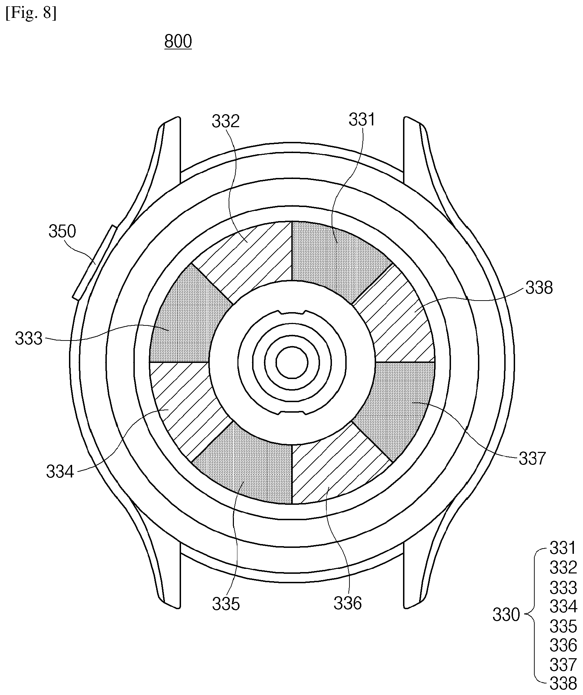

Hereinafter, first combining of rear-side electrodes will be described with reference to FIG. 8. FIG. 8 is a planar view 800 illustrating a wearable electronic device according to an embodiment.

Referring to FIG. 8, the first rear-side electrode 331, the third rear-side electrode 333, the fifth rear-side electrode 335, and the seventh rear-side electrode 337 may be connected to the biometric signal processing circuit as the first electrode set, and the second rear-side electrode 332, the fourth rear-side electrode 334, the sixth rear-side electrode 336, and the eighth rear-side electrode 338 may be connected to the biometric signal processing circuit as the second electrode set.

In the first combining of rear-side electrodes, the sum of the number of rear-side electrodes 331, 333, 335, and 337 of the first electrode set and the number of rear-side electrodes 332, 334, 336, and 338 of the second electrode set may be equal to the number of the plurality of rear-side electrodes 330. The rear-side electrodes 331, 333, 335, and 337 of the first electrode set and the rear-side electrodes 332, 334, 336, and 338 of the second electrode set may be alternately arranged. The combining of rear-side electrodes illustrated in FIG. 8 is an example, and arrangement of the first electrode set and the second electrode set is not limited thereto.

The wearable electronic device according to an embodiment may determine the arrangement of the first electrode set and the second electrode set to be connected to the sensor driving circuit (e.g., the sensor driving circuit 360 of FIG. 3) based on the light quantity information and/or DC offset information described above with reference to FIGS. 3 to 5. That is, the wearable electronic device according to an embodiment may determine, based on the light quantity information and/or the DC offset information, a first region in which the first electrode set (e.g., the rear-side electrodes 331, 333, 335, and 337) is to be located and a second region in which the second electrode set (e.g., the rear-side electrodes 332, 334, 336, and 338) is to be located.

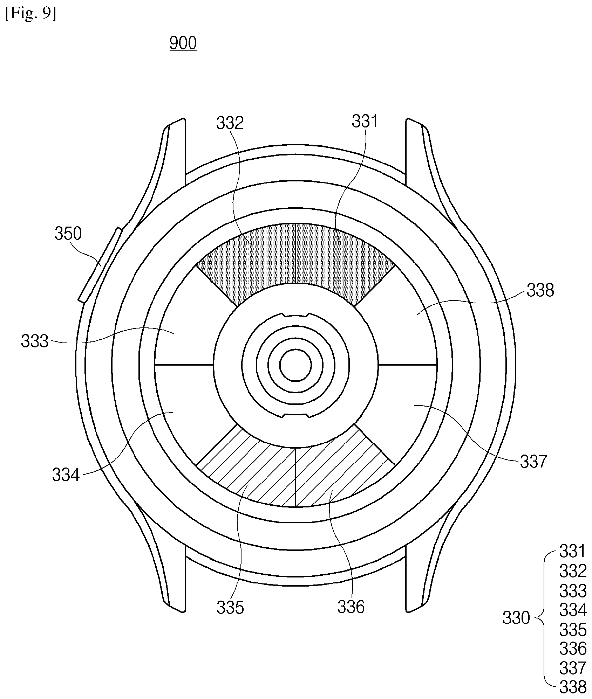

Hereinafter, second combining of rear-side electrodes will be described with reference to FIG. 9. FIG. 9 is a planar view 900 illustrating a wearable electronic device according to an embodiment.

Referring to FIG. 9, the first rear-side electrode 331 and the second rear-side electrode 332 may be connected to the biometric signal processing circuit as the first electrode set, and the fifth rear-side electrode 335 and the sixth rear-side electrode 336 may be connected to the biometric signal processing circuit as the second electrode set. The third rear-side electrode 333, the fourth rear-side electrode 334, the seventh rear-side electrode 337, and the eighth rear-side electrode 338 may not be connected to the biometric signal processing circuit.

In the second combining of rear-side electrodes, the sum of the number of rear-side electrodes 331 and 332 of the first electrode set and the number of rear-side electrodes 335 and 336 of the second electrode set may be less than the number of the plurality of rear-side electrodes 330. The combining of rear-side electrodes illustrated in FIG. 9 is an example, and arrangement of the first electrode set and the second electrode set is not limited thereto.

The wearable electronic device according to an embodiment may determine the arrangement of the first electrode set and the second electrode set to be connected to the sensor driving circuit (e.g., the sensor driving circuit 360 of FIG. 3) based on the light quantity information and/or DC offset information. That is, the wearable electronic device according to an embodiment may determine, based on the light quantity information and/or the DC offset information, a first region in which the first electrode set (e.g., the rear-side electrodes 331 and 332) is to be located and a second region in which the second electrode set (e.g., the rear-side electrodes 335 and 336) is to be located.

The electronic device according to certain embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that certain embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B", "at least one of A and B", "at least one of A or B", "A, B, or C", "at least one of A, B, and C", and "at least one of A, B, or C" may include any one of, or all possible combinations of the items enumerated together in a corresponding one of the phrases. As used herein, such terms as "1st" and "2nd", or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with", "coupled to", "connected with", or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic", "logic block", "part", or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor(e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a compiler or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to certain embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to certain embodiments, each component (e.g., a module or a program) of the above-described components may include a single entity or multiple entities. According to certain embodiments, one or more of the above-described components may be omitted, or one or more other components may be added. Alternatively or additionally, a plurality of components (e.g., modules or programs) may be integrated into a single component. In such a case, according to certain embodiments, the integrated component may still perform one or more functions of each of the plurality of components in the same or similar manner as they are performed by a corresponding one of the plurality of components before the integration. According to certain embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable electronic device (210), comprising:
a housing (410) including a front plate and a rear plate (411);
a light source (310) disposed on the rear plate (411) and configured to emit light;
a plurality of photodetectors (320) disposed on the rear plate (411) and configured to detect light;
three or more rear-side electrodes (330) disposed on the rear plate (411);
a biometric signal processing circuit disposed within the housing (410); and
a processor (120) disposed within the housing (410) and operatively connected to the biometric signal processing circuit,
wherein the processor (120) is configured to:
based on the light detected through the plurality of photodetectors (320), obtain first information including light quantity information,
indicating whether at least one of the three or more rear-side electrodes (330) is floated from a user's body,
based on the first information, select at least two of the three or more rear-side electrodes (330) that are being contacted with the user's body,
execute an electrode combining operation (503, 506) by setting at least one rear-side electrode (330) among the selected rear-side electrodes as a first electrode set, and setting at least one other rear-side electrode (330) among the selected rear-side electrodes as a second electrode set, and
detect biometric information using the first electrode set and the second electrode set.

2. The wearable electronic device (210) of claim 1, wherein the processor is further configured to:
select the two or more rear-side electrodes by excluding one or more rear-side electrodes (330) that is disposed within a predetermined first distance from a photodetector, in which a light quantity exceeding a maximum light quantity threshold is detected, among the plurality of photodetectors (320).

3. The wearable electronic device (210) of claim 1, wherein all of the three or more rear-side electrodes (330) are included when executing the electrode combining operation (503, 506).

4. The wearable electronic device (210) of claim 1, where some of the three or more rear-side electrodes (330) less than an entirety thereof are included when executing the electrode combining operation (503, 506).

5. The wearable electronic device (210) of claim 1, further comprising:
an other-side electrode (350) located on one side of the housing (410) and electrically connected to the biometric signal processing circuit,
wherein the processor (120) is configured to obtain the biometric information through at least two of the first electrode set, the second electrode set, and the other-side electrode (350).

6. A wearable electronic device (210), comprising:
a housing (410) including a front plate and a rear plate (411);
three or more rear-side electrodes (330) disposed on the rear plate (411);
a biometric signal processing circuit disposed within the housing (410);
an other-side electrode (350) located on one side of the housing (410) and electrically connected to the biometric signal processing circuit; and
a processor (120) disposed within the housing (410),
wherein the processor (120) is configured to:
detect a DC offset using at least one of the three or more rear-side electrodes (330) and the other-side electrode (350),
select at least two rear-side electrodes (330) from among the three or more rear-side electrodes (330) based on the DC offset,
operably connect the at least two selected rear-side electrodes (330) to the biometric signal processing circuit; and
obtain biometric information using the at least two rear-side electrodes (330) connected to the biometric signal processing circuit and the other-side electrode (350).

7. The wearable electronic device (210) of claim 6, wherein at least one of the at least two rear-side electrodes (330) operably connected to the biometric signal processing circuit is included in a first electrode set, and
wherein other rear-side electrodes (330) are included in a second electrode set.

8. The wearable electronic device (210) of claim 7, wherein the processor (120) is configured to adjust a number of rear-side electrodes (330) included in the first electrode set based on the DC offset.

9. The wearable electronic device (210) of claim 8, wherein the processor (120) is configured to:
reduce the number of rear-side electrodes (330) included in the first electrode set when the DC offset exceeds a first voltage value, and
increase the number of rear-side electrodes (330) included in the first electrode set when the DC offset is less than a second voltage value.

10. The wearable electronic device (210) of claim 7, wherein the processor (120) is configured to obtain the biometric information using the first electrode set, the second electrode set, and the other-side electrode (350) when the DC offset has a value within a first predetermined threshold range.

## Patentansprüche

1. Tragbare elektronische Vorrichtung (210), umfassend:
ein Gehäuse (410), das eine vordere Platte und eine hintere Platte (411) enthält;
eine Lichtquelle (310), die auf der hinteren Platte (411) angeordnet und konfiguriert ist, um Licht zu emittieren;
eine Vielzahl von Fotodetektoren (320), die auf der hinteren Platte (411) angeordnet und konfiguriert sind, um Licht zu erfassen;
drei oder mehr auf der hinteren Platte (411) angeordnete rückseitige Elektroden (330);
eine im Gehäuse (410) angeordnete Schaltung zur Verarbeitung biometrischer Signale; und
einen Prozessor (120), der innerhalb des Gehäuses (410) angeordnet und betriebsfähig mit der Schaltung zur Verarbeitung biometrischer Signale verbunden ist,
wobei der Prozessor (120) konfiguriert ist zum:
basierend auf dem von der Vielzahl von Fotodetektoren (320) erfassten Licht Erhalten erster Informationen, die Lichtmengen-Informationen umfassen, die angeben, ob mindestens eine der drei oder mehr rückseitigen Elektroden (330) gegenüber dem Körper eines Benutzers in Schwebezustand versetzt wird,
basierend auf den ersten Informationen Auswählen von mindestens zwei der drei oder mehr rückseitigen Elektroden (330), die mit dem Körper des Benutzers in Kontakt gebracht werden,
Durchführen eines Elektrodenkombinationsvorgangs (503, 506) durch Festlegen mindestens einer rückseitigen Elektrode (330) unter den ausgewählten rückseitigen Elektroden als erster Elektrodensatz und Festlegen mindestens einer weiteren rückseitigen Elektrode (330) unter den ausgewählten rückseitigen Elektroden als zweiter Elektrodensatz, und
Erfassen biometrischer Informationen unter Verwendung des ersten Elektrodensatzes und des zweiten Elektrodensatzes.

2. Tragbare elektronische Vorrichtung nach Anspruch 1, wobei der Prozessor ferner konfiguriert ist zum:
Auswählen der zwei oder mehr rückseitigen Elektroden durch Ausschließen einer oder mehrerer rückseitiger Elektroden (330), die innerhalb eines vorbestimmten ersten Abstands von einem Fotodetektor angeordnet sind, bei dem unter der Vielzahl von Fotodetektoren (320) eine Lichtmenge erfasst wird, die einen maximalen Lichtmengenschwellenwert überschreitet.

3. Tragbare elektronische Vorrichtung (210) nach Anspruch 1, wobei beim Durchführen des Elektrodenkombinationsvorgangs (503, 506) alle der drei oder mehr rückseitigen Elektroden (330) enthalten sind.

4. Tragbare elektronische Vorrichtung (210) nach Anspruch 1, wobei einige der drei oder mehr rückseitigen Elektroden (330), die weniger sind als deren Gesamtheit, beim Ausführen des Elektrodenkombinationsvorgangs (503, 506) enthalten sind.

5. Tragbare elektronische Vorrichtung (210) nach Anspruch 1, ferner umfassend:
eine andersseitige Elektrode (350), die sich auf einer Seite des Gehäuses (410) befindet und elektrisch mit der Schaltung zur Verarbeitung biometrischer Signale verbunden ist,
wobei der Prozessor (120) konfiguriert ist, um die biometrischen Informationen über mindestens zwei von dem ersten Elektrodensatz, dem zweiten Elektrodensatz und der andersseitigen Elektrode (350) zu erhalten.

6. Tragbare elektronische Vorrichtung (210), umfassend:
ein Gehäuse (410), das eine vordere Platte und eine hintere Platte (411) enthält;
drei oder mehr auf der hinteren Platte (411) angeordnete rückseitige Elektroden (330);
eine im Gehäuse (410) angeordnete Schaltung zur Verarbeitung biometrischer Signale;
eine andersseitige Elektrode (350), die sich auf einer Seite des Gehäuses (410) befindet und elektrisch mit der Schaltung zur Verarbeitung biometrischer Signale verbunden ist; und
einen im Gehäuse (410) angeordneten Prozessor (120),
wobei der Prozessor (120) konfiguriert ist zum:
Erfassen eines Gleichstrom-Offsets unter Verwendung mindestens einer der drei oder mehr rückseitigen Elektroden (330) und der andersseitigen Elektrode (350),
Auswählen von mindestens zwei rückseitigen Elektroden (330) aus den drei oder mehr rückseitigen Elektroden (330) basierend auf dem Gleichstrom-Offset,
betriebsfähiges Verbinden der mindestens zwei ausgewählten rückseitigen Elektroden (330) mit der Schaltung zur Verarbeitung biometrischer Signale; und
Erhalten biometrischer Informationen unter Verwendung der mindestens zwei rückseitigen Elektroden (330), die mit der Schaltung zur Verarbeitung biometrischer Signale verbunden sind, und der andersseitigen Elektrode (350).

7. Tragbare elektronische Vorrichtung (210) nach Anspruch 6, wobei mindestens eine der mindestens zwei rückseitigen Elektroden (330), die betriebsfähig mit der Schaltung zur Verarbeitung biometrischer Signale verbunden sind, in einem ersten Elektrodensatz enthalten ist, und
wobei weitere rückseitige Elektroden (330) in einem zweiten Elektrodensatz enthalten sind.

8. Tragbare elektronische Vorrichtung (210) nach Anspruch 7, wobei der Prozessor (120) so konfiguriert ist, dass er eine Anzahl der im ersten Elektrodensatz enthaltenen rückseitigen Elektroden (330) basierend auf dem Gleichstrom-Offset anpasst.

9. Tragbare elektronische Vorrichtung (210) nach Anspruch 8, wobei der Prozessor (120) konfiguriert ist zum:
Verringern der Anzahl von in dem ersten Elektrodensatz enthaltenen rückseitigen Elektroden (330), wenn der Gleichstrom-Offset einen ersten Spannungswert überschreitet, und
Erhöhen der Anzahl von im ersten Elektrodensatz enthaltenen rückseitigen Elektroden (330), wenn der Gleichstrom-Offset kleiner als ein zweiter Spannungswert ist.

10. Tragbare elektronische Vorrichtung (210) nach Anspruch 7, wobei der Prozessor (120) so konfiguriert ist, dass er die biometrischen Informationen unter Verwendung des ersten Elektrodensatzes, des zweiten Elektrodensatzes und der andersseitigen Elektrode (350) erhält, wenn der Gleichstrom-Offset einen Wert innerhalb eines ersten vorgegebenen Schwellenwertbereichs aufweist.

## Revendications

1. Dispositif électronique portable (210), comprenant :
un boîtier (410) comprenant une plaque frontale et une plaque arrière (411) ;
une source lumineuse (310) disposée sur la plaque arrière (411) et configurée pour émettre de la lumière ;
une pluralité de photodétecteurs (320) disposés sur la plaque arrière (411) et configurés pour détecter la lumière ;
au moins trois électrodes de côté arrière (330) disposées sur la plaque arrière (411) ;
un circuit de traitement de signaux biométriques disposé à l'intérieur du boîtier (410) ; et
un processeur (120) disposé à l'intérieur du boîtier (410) et connecté de manière opérationnelle au circuit de traitement de signaux biométriques,
dans lequel le processeur (120) est configuré pour :
en se basant sur la lumière détectée par la pluralité de photodétecteurs (320), obtenir des premières informations comprenant des informations de quantité de lumière indiquant si au moins une des trois électrodes de côté arrière (330) ou plus est flottante par rapport à un corps d'un utilisateur,
en se basant sur les premières informations, sélectionner au moins deux des trois électrodes de coté arrière (330) ou plus qui sont en contact avec le corps de l'utilisateur,
effectuer une opération de combinaison d'électrodes (503, 506) en définissant au moins une électrode de côté arrière (330) parmi les électrodes de côté arrière sélectionnées comme premier ensemble d'électrodes, et définir au moins une autre électrode de côté arrière (330) parmi les électrodes de côté arrière sélectionnées comme deuxième ensemble d'électrodes, et
détecter des informations biométriques à l'aide du premier ensemble d'électrodes et du deuxième ensemble d'électrodes.

2. Dispositif électronique portable (210) selon la revendication 1, dans lequel le processeur est en outre configuré pour :
sélectionner au moins deux électrodes de côté arrière en excluant une ou plusieurs électrodes de côté arrière (330) disposées à une première distance prédéterminée d'un photodétecteur, dans lequel une quantité de lumière dépassant un seuil maximal de quantité de lumière est détectée, parmi la pluralité de photodétecteurs (320).

3. Dispositif électronique portable (210) selon la revendication 1, dans lequel toutes des trois électrodes ou plus de côté arrière (330) sont comprises lors de l'exécution de l'opération de combinaison d'électrodes (503, 506).

4. Dispositif électronique portable (210) selon la revendication 1, dans lequel quelques-unes des trois électrodes de côté arrière (330) ou plus, moins que la totalité de celles-ci, sont comprises lors de l'exécution de l'opération de combinaison d'électrodes (503, 506).

5. Dispositif électronique portable (210) de la revendication 1, comprenant en outre :
une électrode d'autre côté (350) située d'un côté du boîtier (410) et reliée électriquement au circuit de traitement de signaux biométriques,
dans lequel le processeur (120) est configuré pour obtenir les informations biométriques par l'intermédiaire d'au moins deux du premier ensemble d'électrodes, le deuxième ensemble d'électrodes et l'électrode d'autre côté (350).

6. Dispositif électronique portable (210), comprenant :
un boîtier (410) comprenant une plaque frontale et une plaque arrière (411) ;
au moins trois électrodes de côté arrière (330) disposées sur la plaque arrière (411) ;
un circuit de traitement de signaux biométriques disposé à l'intérieur du boîtier (410) ;
une électrode d'autre côté (350) située d'un côté du boîtier (410) et reliée électriquement au circuit de traitement de signaux biométriques ; et
un processeur (120) disposé à l'intérieur du boîtier (410),
dans lequel le processeur (120) est configuré pour :
détecter un décalage en courant continu à l'aide d'au moins une des trois électrodes ou plus de côté arrière (330) et de l'électrode d'autre côté (350),
sélectionner au moins deux électrodes de coté arrière (330) parmi les trois électrodes de côté arrière (330) ou plus, en se basant sur le décalage en courant continu,
relier de manière opérationnelle les au moins deux électrodes de côté arrière sélectionnées (330) au circuit de traitement de signaux biométriques ; et
obtenir des informations biométriques à l'aide des au moins deux électrodes de côté l'arrière (330) reliées au circuit de traitement de signaux biométriques et de l'électrode d'autre côté (350).

7. Dispositif électronique portable (210) selon la revendication 6, dans lequel au moins l'un des au moins deux électrodes arrière (330) connectés de manière opérationnelle au circuit de traitement de signaux biométriques est comprise dans un premier ensemble d'électrodes, et
dans lequel d'autres électrodes de côté arrière (330) sont comprises dans un ensemble d'électrodes.

8. Dispositif électronique portable (210) selon la revendication 7, dans lequel le processeur (120) est configuré pour ajuster un nombre d'électrodes de côté arrière (330) comprises dans le premier ensemble d'électrodes en se basant sur le décalage en courant continu.

9. Dispositif électronique portable (210) selon la revendication 8, dans lequel le processeur (120) est configuré pour :
réduire le nombre d'électrodes de côté arrière (330) comprises dans le premier ensemble d'électrodes lorsque le décalage en courant continu dépasse une première valeur de tension, et
augmenter le nombre d'électrodes de côté arrière (330) comprises dans le premier ensemble d'électrodes lorsque le décalage en courant continu est inférieur à une deuxième valeur de tension.

10. Dispositif électronique portable (210) selon la revendication 7, dans lequel le processeur (120) est configuré pour obtenir les informations biométriques à l'aide du premier ensemble d'électrodes, du deuxième ensemble d'électrodes et de l'électrode d'autre côté (350) lorsque le décalage en courant continu présente une valeur comprise dans une première plage de seuil prédéterminée.
